# EUROPEAN PATENT SPECIFICATION

(11) **EP 3 221 697 B1**
(45) Date of publication and mention of the grant of the patent: **23.11.2022**
(21) Application number: 15861319.0
(22) Date of filing: 13.11.2015
(51) Int. Cl.: C12Q 1/6883, A61P 9/00, A61P 9/10

(54) **METHODS FOR MONITORING EFFICACY OF ALLOGENEIC MESENCHYMAL STEM CELL THERAPY IN A SUBJECT**
VERFAHREN ZUR ÜBERWACHUNG DER WIRKSAMKEIT EINER ALLOGENEN MESENCHYMALEN STAMMZELLENTHERAPIE BEI EINER PERSON
PROCÉDÉS DE SURVEILLANCE DE L'EFFICACITÉ DU CELLULE SOUCHE MÉSENCHYMATEUSES ALLOGÉNIQUES TRAITEMENT CHEZ UN PATIENT

(30) Priority: 17.11.2014 US 201462080984 P
(43) Date of publication of application: 27.09.2017
(73) Proprietor: University of Miami, Miami, FL 33136 (US)
(72) Inventor: HARE, Joshua, M., Miami Beach, FL 33141 (US); PREMER, Courtney, North Miami, FL 33181 (US); BLUM, Arnon, 36001 Nofit (IL)
(74) Representative: Inspicos P/S
(86) International application number: PCT/US2015/060624
(87) International publication number: WO 2016/081309

(56) References cited:
- US-A1- 2011 104 124
- US-A1- 2012 269 785
- NARITA TAKUYA ET AL: "Bone marrow-derived mesenchymal stem cells for the treatment of heart failure", HEART FAILURE REVIEWS, KLUWER ACADEMIC PUBLISHERS, DORDRECHT, NL, vol. 20, no. 1, 27 May 2014 (2014-05-27), pages 53-68, XP035417931, ISSN: 1382-4147, DOI: 10.1007/S10741-014-9435-X [retrieved on 2014-05-27]
- IMANISHI Y ET AL: "Allogenic mesenchymal stem cell transplantation has a therapeutic effect in acute myocardial infarction in rats", JOURNAL OF MOLECULAR AND CELLULAR CARDIOLOGY, ACADEMIC PRESS, GB, vol. 44, no. 4, 1 April 2008 (2008-04-01), pages 662-671, XP022640584, ISSN: 0022-2828, DOI: 10.1016/J.YJMCC.2007.11.001 [retrieved on 2007-11-13]
- ALYNE FELIX ET AL: "Remodeling of the thoracic aorta after bone marrow cell transplantation", INTERNATIONAL JOURNAL OF CLINICAL AND EXPERIMENTAL PATHOLOGY, vol. 7, no. 9, 15 August 2014 (2014-08-15) , pages 5527-5537, XP055444572, US ISSN: 1936-2625
- RASTOGI S ET AL: "Effects of intravenous injections of hypoxia-conditioned bone marrow-derived stem cell medium in dogs with heart failure", EUROPEAN JOURNAL OF HEART FAILURE SUPPLEMENTS, ELSEVIER, vol. 7, no. 1, 1 June 2008 (2008-06-01), page 114, XP025768719, ISSN: 1567-4215, DOI: 10.1016/S1567-4215(08)60316-1 [retrieved on 2008-06-01]
- MOON HYUNG-HO ET AL: "MSC-based VEGF gene therapy in rat myocardial infarction model using facial amphipathic bile acid-conjugated polyethyleneimine", BIOMATERIALS, vol. 35, no. 5, 23 November 2013 (2013-11-23), pages 1744-1754, XP028801323, ISSN: 0142-9612, DOI: 10.1016/J.BIOMATERIALS.2013.11.019
- PREMER COURTNEY ET AL: "Intracardiac Mesenchymal Stem Cells Restore Endothelial Function in Heart Failure by Stimulating the Release of Endothelial Progenitor Cells", CIRCULATION (BALTIMORE), LIPPINCOTT WILLIAMS & WILKINS, US, vol. 130, no. Suppl. 2, 25 November 2014 (2014-11-25), page 18801, XP009503727, ISSN: 0009-7322
- FELIX ET AL.: 'Remodeling of the thoracic aorta after bone marrow cell transplantation.' INTERNATIONAL JOURNAL OF CLINICAL AND EXPERIMENTAL PATHOLOGY vol. 7, no. 9, 15 August 2014, pages 5527 - 5537, XP055444572
- ICHIM THOMAS E ET AL: "Placental mesenchymal and cord blood stem cell therapy for dilated cardiomyopathy", REPRODUCTIVE BIOMEDICINE ONLINE,, vol. 16, no. 6, 1 June 2008 (2008-06-01), pages 898-905, XP009180675, ISSN: 1472-6491, DOI: 10.1016/S1472-6483(10)60159-9
- PSALTIS P J ET AL: "Reparative Effects of Allogeneic Mesenchymal Precursor Cells Delivered Transendocardially in Experimental Nonischemic Cardiomyopathy", JACC: CARDIOVASCULAR INTERVENTIONS, ELSEVIER, AMSTERDAM, NL, vol. 3, no. 9, 1 September 2010 (2010-09-01), pages 974-983, XP027290326, ISSN: 1936-8798 [retrieved on 2010-09-01]
- Joshua M. Hare ET AL: "Comparison of Allogeneic vs Autologous Bone Marrow-Derived Mesenchymal Stem Cells Delivered by Transendocardial Injection in Patients With Ischemic Cardiomyopathy", JAMA THE JOURNAL OF THE AMERICAN MEDICAL ASSOCIATION, vol. 308, no. 22, 12 December 2012 (2012-12-12), page 2369, XP55254332, US ISSN: 0098-7484, DOI: 10.1001/jama.2012.25321

## Description

### FIELD OF THE INVENTION

The present disclosure is directed to materials and methods for treating endothelial dysfunction in a human subject and for monitoring efficacy of allogeneic mesenchymal stem cell therapy in a subject suffering from a cardiovascular disorder.

### BACKGROUND

Heart failure (HF) remains a leading cause of morbidity and mortality in the United States¹. The failing circulation is characterized not only by depressed cardiac function, but also by endothelial dysfunction². Endothelial dysfunction produces increased systemic vascular resistance, which augments stress on the failing heart, and contributes to HF symptomology^{3,4}, Endothelial dysfunction is also a crucial component of the pathophysiology of numerous cardiovascular (CV) disorders and manifests in patients with CV risk factors such as atherosclerosis, hypertension, and diabetes mellitus^{5,6}. Moreover, endothelial progenitor cells (EPCs) regulate the health of the vasculature by incorporating into the endothelium, replacing injured endothelial cells, and secreting angiogenic factors that activate mature endothelial cells^{7,8} . Notably, HF patients have decreased circulating EPC levels and bioactivity⁷. Narita Takuya et al (Heart Failure Reviews, Kluwer Academic Publishers, Vol. 20(1), p. 53-68, 27-05-2014) relates to bone marrow-derived mesenchymal stem cell (MSC)-based therapy for heart failure. It mentions that if allogeneic MSCs can survive after transplantation to a similar degree to autologous MSCs, it would be of great advantage. Narita et al, however, indicates that further investigations are needed to elucidate whether allogeneic MSCs are really useful as donor for cell therapy for heart failure.

### SUMMARY

A method of monitoring efficacy of a therapy for endothelial dysfunction is provided. In one aspect, the method comprises determining levels of Vascular Endothelial Growth Factor (VEGF) and endothelial progenitor cells (EPCs) in a first sample from the subject before administration of a therapy for endothelial dysfunction; and determining levels of VEGF and EPCs in a second sample from the subject after administration of the therapy for endothelial dysfunction; wherein a decreased level of VEGF and an increased level of EPCs in the second sample compared to levels of VEGF and EPCs in the first sample is indicative of effective treatment of the endothelial dysfunction. In some embodiments, the therapy for endothelial dysfunction comprises mesenchymal stem cell therapy. In some embodiments, the mesenchymal stem cell therapy comprises the administration of allogeneic mesenchymal stem cells to the subject.

Described herein, but not part of the invention is a method of monitoring efficacy of a therapy for endothelial dysfunction comprising determining levels of Vascular Endothelial Growth Factor (VEGF) and endothelial progenitor cells (EPCs) in a sample from the subject after administration of the therapy for endothelial dysfunction, and comparing the levels of VEGF and EPC to a predetermined criterion, e.g., levels of VEGF and EPC from healthy subjects. A decreased level of VEGF (such that the level of VEGF in the sample is commensurate with a level of VEGF in healthy subjects) and an increased level of EPCs (such that the level of EPCs is commensurate with a level of EPCs in healthy subjects) in the sample compared to the predetermined criterion is indicative of effective treatment of the endothelial dysfunction. Any therapy for endothelial cell dysfunction is contemplated herein; in one aspect, the therapy is a cell-based therapy, such as administration of stem cells (e.g., mesenchymal stem cells).

Also described herein is a method of monitoring efficacy of a mesenchymal stem cell therapy in a subject suffering from a cardiovascular disorder comprising determining a level of Vascular Endothelial Growth Factor (VEGF) in a sample from the subject after administration of the stem cell therapy, and comparing the level of VEGF to a predetermined criterion, e.g., level of VEGF from healthy subjects. A decreased level of VEGF in the sample such that it is commensurate with a level of VEGF in healthy subjects is indicative of effective stem cell therapy.

The steps of determining the level of VEGF and/or EPCs in the sample is performed by any means, such as those known in the art and described below. For example, the determining the level of VEGF in a sample entails quantifying the amount of VEGF protein in the sample, quantifying the amount of VEGF DNA or mRNA also is contemplated. In some embodiments, the method comprises isolating EPCs from the sample, culturing the EPCs for at least 24 hours, and determining the number of colony forming units (CFUs).

In some embodiments, the sample is a blood sample or serum sample.

Described herein, but not part of the invention is a method of identifying endothelial dysfunction in a subject comprising determining a level of Vascular Endothelial Growth Factor (VEGF) in a sample from the subject; and determining a level of endothelial progenitor cells (EPCs) in the sample from the subject, wherein an elevated level of VEGF in the sample and a decreased level of EPC in the sample is indicative of endothelial dysfunction in the subject

An aspect which is not part of the invention is a method of treating endothelial dysfunction in a subject in need thereof comprising administering to a subject allogeneic mesenchymal stem cells (MSCs) in an amount effect to decrease circulating levels of VEGF and increase levels of EPCs in the subject, thereby treating the endothelial dysfunction. In some embodiments, the subject is suffering from a cardiovascular disorder such as, for example, heart failure, idiopathic dilated cardiomyopathy and ischemic cardiomyopathy. In various embodiments, the method improves vascular tone.

The mesenchymal stem cells are administered according to any suitable method, including those known in the art. In some embodiments, the cells are administered locally. In some embodiments, the cells are administered by transendocardial injection.

The mesenchymal stem cells are optionally administered in an amount ranging from about 20 million to about 100 million cells. In some embodiments, the mesenchymal stem cells are administered in an amount ranging from about 20 million to about 30 million cells, or about 20 millions to about 40 million cells, or about 20 million to about 50 million cells, or about 20 million to about 60 million cells, or about 20 million to about 70 million cells, or about 20 million to about 80 million cells, or about 20 million to about 90 million cells, or about 30 million to about 50 million cells, or about 30 million to about 70 million, or about 30 million to about 90 million cells, or about 50 million to about 100 million cells. In some embodiments, about 20 million, or about 25 million, or about 30 million, or about 35 million, or about 40 million, or about 45 million, or about 50 million, or about 55 million, or about 50 million, or about 55 million, or about 60 million, or about 65 million, or about 70 million, or about 75 million, or about 80 million, or about 85 million, or about 90 million, or about 95 million, or about 100 million mesenchymal stem cells are administered to the subject.

In some embodiments, administration of the mesenchymal stem cells to the subject results in an increase in flow-mediated vasodilation (FMD) in the subject of at least 3%.

In any of the methods described herein, the subject is a human subject.

### BRIEF DESCRIPTION OF THE FIGURES

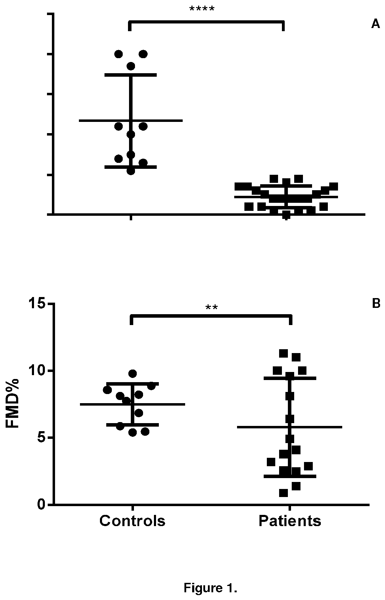

Figure 1. Endothelial function in patients with heart failure, including dilated and ischemic cardiomyopathy. Figure 1A shows that patients (n=22) in the study had impaired EPC-CFUs compared to healthy controls (n=10, ^{∗∗∗∗}P<0.001). Figure 1B shows that patients (n=22) in the study had reduced FMD% compared to healthy controls (n=10, ^{∗∗}P=0.0017).

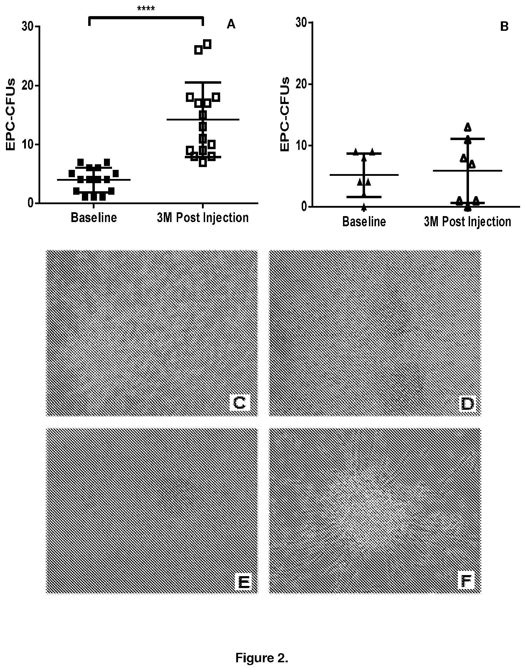

Figure 2. Endothelial colony forming units in heart failure patients treated with either allogeneic or autologous mesenchymal stem cells (MSCs). Figure 2A shows that patients treated with allogeneic MSCs had a significant improvement in EPC-CFUs 3 months post treatment (n=15, ^{∗∗∗∗}P<0.001). Figure 2B shows that patients treated with autologous MSCs had no change in EPC-CFUs post treatment (n=7). Figures 2C and 2E show representative EPC-CFUs plated on fibronectin for 5 days before MSC administration (magnification 20x). Figures 2D and 2F shows representative EPC-CFYs plated on fibronectin for 5 days after MSC administration (magnification 20x).

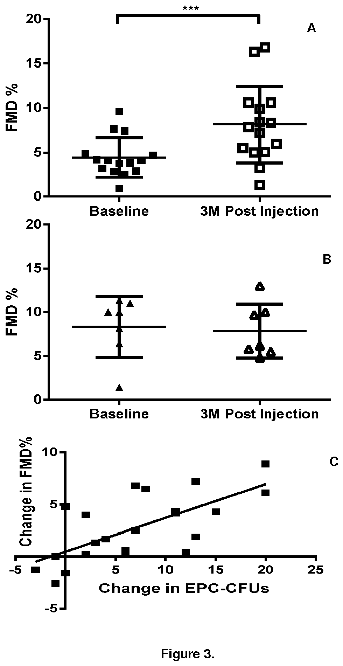

Figure 3. Flow mediated vasodilation (FMD) measurements before and after mesenchymal stem cell (MSC) treatment. Figure 3A shows that patients treated with allogeneic MSCs had an increase in FMD% 3 months post injection (n=15, ^{∗∗∗}P<0.001). Figure 3B shows that patients treated with autologous MSCs had no significant difference in FMD% 3 months post injection (n=7, P=NS). Figure 3C shows that correlation between the absolute change in FMD% and the absolute change in EPF-CFUs from baseline to 3 months post MSC injection in all patients (P<0.001, R=0.684).

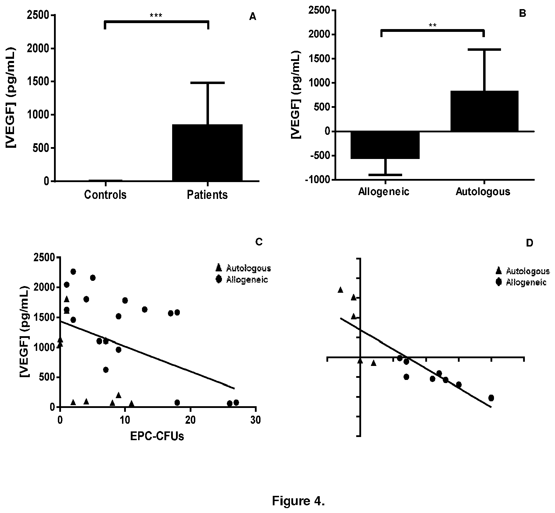

Figure 4. Serum VEGF concentration in patients and controls. Figure 4A demonstrates that patients (n=14) had a higher level of circulating VEGF compared to controls (n=9) at baseline (P=0.0009). Figure 4B demonstrates that patients who received allogeneic MSCs (n=9) had a decrease in VEGF serum levels post injection (Δ-547.5±350.8 pg/mL), while patients who received autologous MSCs (n=5) had an increase in serum VEGF post injection (Δ814.1±875.8), and there was a difference between groups (^{∗∗}P=0.0012). Figure 4C shows that there is a correlation between EPC-CFUs and serum VEGF in patients at both baseline and 3 months post MSC treatment (R=-0.421, P=0.026). Figure 4D shows that the change in EPC-CFUs from baseline to 3 months post treatment strongly correlated with the change in VEGF (R=-0.863, P<0.001).

### DETAILED DESCRIPTION

Herein described is that increased circulating levels of Vascular Endothelial Growth Factor (VEGF) in combination with decreased levels of endothelial progenitor cells (EPCs), is indicative of endothelial dysfunction in a subject, and reduced levels of VEGF and increased levels of EPCs are indicative of effectiveness of therapy for endothelial dysfunction, vascular tone, and cardiovascular disease. Data provided herein demonstrated that the administration of allogeneic mesenchymal stem cells (MSCs) to subjects suffering from heart failure stimulated EPC bioactivity and restored flow-mediated vasodilation towards normal. The ability of allogeneic MSCs greatly exceeded that of autologous MSCs in restoring endothelial function, enhancing EPC CFU formation, and suppressing VEGF levels. Together these findings offer major new clinical insights into the bioactivity of MSCs, suggest a novel therapeutic principle for disorders characterized by endothelial dysfunction, and provide useful diagnostic markers for detecting and measuring successful treatment.

MSCs are adult stem cells that are prototypically found in bone marrow and have the capacity to differentiate into multiple cell types. They stimulate the proliferation and differentiation of endogenous precursor cells and play a crucial role in maintaining stem cell niches²⁷. In addition, MSCs secrete paracrine factors which participate in angiogenesis, cardiomyogenesis, neovascularization, stimulation of other endogenous stem cells, and regulation of the immune system^{28,29}. While MSCs are known to stimulate cardiac precursor cells and cell cycle activity in the heart, their role in stimulating other endogenous precursor populations has heretofore been unknown. The data provided herein establish that MSCs stimulate endogenous EPC activation, increasing the number and quality of functional EPCs. These findings suggest that augmentation of EPCs may represent a novel mechanism of action by which MSCs exert favorable biological effects.

Over the last decade, there has been an emerging interest in the use of MSCs in CV disorders³⁰. Clinical trials have demonstrated a major safety profile for MSC administration, and suggested efficacy in patients with HF³⁰⁻³²; however, underlying mechanism(s) of action continue to be vigorously debated. The instant finding that allogeneic MSC injections in patients with both ischemic and non-ischemic HF results in an improvement in endothelial function specifically by restoring EPC function, FMD, and reducing VEGF levels towards normal offers a major new insight into the mechanisms of action of MSCs. In the study population, increased serum VEGF correlated with diminished EPC-CFUs, consistent with the idea that VEGF plays a compensatory role, a finding also reported in patients with cerebral aneurysm²⁶. The findings provided herein establish that allogeneic MSCs can be employed to stimulate EPC bioactivity, improve arterial physiologic vasodilatory responses, and decrease unfavorable cytokine mobilization in patients with CV disease and other disorders associated with endothelial dysfunction.

The data provided herein demonstrate that allogeneic MSCs restored endothelial function in patients to a degree greatly exceeding that of autologous MSCs. In the study described below, all allogeneic stem cell donors were healthy, young donors between the ages of 20 and 35. Patients receiving their own stem cells not only had underlying chronic diseases, but were also older (between the ages of 45 and 75). Autologous MSCs aging may impair the survival, differentiation, and ability to recruit EPCs to areas of damage, ultimately reducing their therapeutic efficacy^{38,39}.

### EXAMPLES

### Methods

*Study Population:* The HF patients were enrolled in POSEIDON-DCM (NCT01392625), "A Phase I/II, Randomized Pilot Study of the Comparative Safety and Efficacy of Transendocardial Injection of Autologous Mesenchymal Stem Cells Versus Allogeneic Mesenchymal Stem Cells in Patients with Nonischemic Dilated Cardiomyopathy" and in TRIDENT (NCT02013674), "The TRansendocardial Stem Cell Injection Delivery Effects on Neomyogenesis Study". In PODEIDON-DCM, patients were randomized to receive by transendocardial delivery either 100 million autologous or allogeneic MSCs. Autologous MSCs were derived from the patient's bone marrow (iliac crest aspiration) 4-6 weeks before cardiac catheterization. In the TRIDENT study, ICM patients were randomized to receive either 20 or 100 million allogeneic MSCs transendocardially. Allogeneic MSCs were manufactured by the University of Miami Cell Manufacturing Program. Healthy subjects (n=10) were enrolled ranging in ages from 22-58 years and both genders. All subjects provided written informed consent and the study was approved by the University of Miami Institutional Review Board.

*Endothelial Colony Forming Units (EPC-CFUs):* Peripheral blood samples were obtained from patients before and 3 months after MSC injection. EPCs were isolated from samples using Ficoll-Paque and 5 million cells were seeded on 6-well Fibronectin-coated dishes (BD biosciences) in CFU-Hill medium (stem cell technologies, cat#05900)17. The non-adherent cells were collected 48 hours later and 1 million cells were seeded on 24-well Fibronectin-coated dishes. On day 5, EPC-CFUs were counted in 5 wells and the average was obtained.

*Flow-Mediated Vasodilation (FMD):* Brachial artery diameter measurements and FMD% were performed in the morning, after an overnight fast. The subjects' right arm was immobilized in an extended position, and the brachial artery was scanned via ultrasound 5-10 cm above the antecubital fossa ^{17,18} . A brachial cuff was then inflated to a supra-systolic pressure (40 to 50 mmHg above systolic pressure) for 5 minutes. Subsequently, the cuff was deflated and the brachial artery diameter was recorded for 3 minutes.

*VEGF ELISA:* Serum vascular endothelium growth factor (VEGF) levels (Invitrogen #KHG0111) were measured in DCM patients at baseline and 6 months after allogeneic (n=6) or autologous (n=5) MSC treatment. In ICM patients (n=4), VEGF was measured at baseline and 3 months after allogeneic MSC treatment. DCM and ICM patients who received allogeneic MSCs were combined and compared to patients who received autologous. Lastly, VEGF was measured in controls (n=9).

*C-reactive Protein:* C-reactive protein (CRP) was evaluated using a nephelometric method in which blood plasma samples were incubated with latex particles coated with CRP monoclonal antibodies. The analytical sensitivity was 0.2mg/L and results were reported in mg/L.

*Statistical Analysis:* To assess the difference between autologous and allogeneic groups, an unpaired, two-tailed T-test was used. To measure the difference before and after treatment in each group, both a paired, two-tailed T-test and a one-way ANOVA was utilized. Correlations were measured using Pearson correlation, assuming a Gaussian distribution. Data are presented as mean and standard deviation of the mean. To determine the difference between HUVEC treatment groups, an unpaired, two-tailed T-test was used.

### Results

*Baseline Characteristics:* A total of 22 patients were analyzed for this study. Fifteen patients received allogeneic MSCs and 7 patients received autologous MSCs. Baseline characteristics of the study subjects are summarized in Table 1. Age and sex were evenly distributed.

**Table 1. Summary of patients (n=22) and healthy controls (n=10). Patients are broken down by etiology and cell treatment: DCM patients receiving allogeneic MSCs (n=9), DCM patients receiving autologous MSCs (n=7) and ICM patients receiving allogeneic MSCs (n=6)..**

| **Characteristic** | **DCM Allogeneic (N=9,27%)** | **DCM Autologous (N=7, 21 %)** | **ICM Allogeneic (N=6,18%)** | **Healthy Controls (N=10, 33%)** |
|---|---|---|---|---|
| **Age** - **yr.** | | | | |
| Median | 60 | 55 | 71 | 44 |
| Range | 45-70 | 48-73 | 65-75 | 22-58 |
| **Male sex- no.** | 8 (89%) | 6 (86%) | 6 (100%) | 7 (70%) |

| **Race/Ethnicity** | | | | |
|---|---|---|---|---|
| White | 8 (89%) | 3 (43%) | 6 (100%) | 7(70%) |
| White/Hispanic | 0 (0%) | 4(57%) | 0(0%) | 3 (30*%*) |
| Black | 1 (11%) | 0(0%) | 0(0%) | 0(0%) |

| **History of CVD** | | | | |
|---|---|---|---|---|
| HF | 2 (22*%*) | 0(0%) | 1 (17%) | 0 (0%) |
| CAD | 1 (11%) | 0(0%) | 6 (100%) | 0(0%) |
| CHF | 0 | 1 (14*%*) | 4 (67%) | 0 (0*%*) |
| **History of Smoking** | 4(44*%*) | 4(57%) | 4 (67%) | 0(0%) |

| **Blood pressure- mmHg (systolic/diastolic)** | | | | |
|---|---|---|---|---|
| Median | 126/77 | 108/65 | 113/70 | 118/67 |
| Range | 108- | 99-142/59-84 | 85-134/53- | 111-130/59- |

| **Cholesterol** | | | | |
|---|---|---|---|---|
| Median | 202 | 175 | 147 | N/A |
| Range | 129-282 | 112-207 | 129-178 | N/A |
| **C-Reactive Protein (mg/mL)** | | | | |
| Median | 0.3 | 0.3 | 0.15 | N/A |
| Range | 0.2-0.4 | 0.2-0.5 | 0.1-0.6 | N/A |

| **Medications** | | | | |
|---|---|---|---|---|
| ASA/NSAIDS | 5 (56%) | 4 (57%) | 5 (83%) | 0 (0*%*) |
| Beta-Blockers | 7 (78*%*) | 7 (100%) | 6 (100%) | 0(0%) |
| ACE | 6 (67%) | 7 (100%) | 6 (100%) | 1 (10%) |
| Diuretics | 8 (89%) | 6 (86%) | 4 (67%) | 0(0%) |
| Statins | 3 (33*%*) | 3 (43*%*) | 6 (100%) | 1 (10%) |
| Antiplatelet | 4 (44*%*) | 2 (29%) | 3 (50%) | 0(0%) |
| Other | 9 (100%) | 7 (100%) | 6 (100%) | 0(0%) |

*EPC-CFUs and FMD in Heart Failure Patients and Healthy Subjects:* Patients with ischemic (n=15) as well as non-ischemic (n=6) cardiomyopathy had endothelial dysfunction at baseline, characterized by a reduced ability to form EPC-CFUs and an impaired FMD response (Figure 1). Specifically, patients had decreased EPC-CFU counts compared to healthy controls (4±3 vs. 25±16, respectively, P<0.001) as well as diminished FMD% (5.6±3.2 vs. 9.2±4, respectively, P=0.0017).

*EPC-CFUs in Heart Failure Patients Treated with MSCs:* Patients were assessed for EPC-CFUs before and 3 months after MSC treatment. Patients who received allogeneic MSCs had a significant improvement in the number of EPC-CFUs post-treatment (Δ10±5, P<0.001, Figure 2A). On the other hand, patients who received autologous MSCs showed no improvement (Δ1±3, P=NS, Figure 2B). Moreover, we compared allogeneic MSC treatment versus autologous MSC treatment, and allogeneic MSCs were superior in stimulating EPC colony formation (P=0.02).

EPC-CFUs were also examined for morphology. EPCs from HF patients had disorganized and incomplete colony formation (Figure 2C and E), resulting in clusters that failed to form functional colonies. Three months after allogeneic MSC treatment, patient colonies were organized and healthy in appearance (Figure 2D and F). These findings suggest that transendocardial MSC therapy stimulates EPC bioactivity in patients with HF of both ischemic and non-ischemic etiology.

*FMD in Heart Failure Patients Treated with MSCs:* All patients were evaluated using brachial artery FMD before and 3 months after MSC injection. Patients who received allogeneic MSCs had a dramatic improvement in FMD% (Δ3.7±3*%*, P=0.001, Figure 3A). In contrast, patients who received autologous MSCs had no improvement and the majority of patients worsened 3 months post-treatment (Δ-0.46±3*%*, Figure 3B).

The difference between treatment with autologous MSCs and allogeneic MSCs was analyzed. There was a striking difference between the two cell types (P=0.005), suggesting that autologous MSCs do not restore endothelial function in this patient population. We also assessed the correlation between EPC-CFUs and FMD% in all patients and found a highly significant correlation between ΔFMD% and ΔEPC-CFUs (P<0.001, R=0.684, Figure 3C).

*Assessment of VEGF in Patients Receiving Autologous and Allogeneic MSCs:* Circulating VEGF levels in patients and healthy control serum was determined. At baseline, patients had profoundly elevated circulating VEGF compared to controls (1130.3±803.3 vs. 2.0±5.9 pg/mL, P<0.001, Figure 4A). Allogeneic MSCs reduced VEGF levels (-547.5±350.8 pg/mL, P=0.002), while patients who received autologous MSCs had an increase in VEGF (814.1±875.8 pg/mL, Figure 4B). Furthermore, there was a significant difference between patients who received allogeneic MSCs versus patients who received autologous MSCs (P=0.0012, Figure 4B).

VEGF levels correlated with EPC-CFUs (P=0.026, R=-0.421, Figure 4C). Even more striking, there was a significant correlation between the change in VEGF and the change in EPC-CFUs from baseline to three months post treatment (R=0.863, P<0.001, Figure 4G). Notably, high levels of VEGF correlated with low levels of EPCs, evidenced by the autologous group. Conversely, lower levels of VEGF correlated with high levels of EPC-CFUs, illustrated by the allogeneic group. Taken together, these data demonstrate MSCs stimulate EPC mobilization and suppress compensatory elevations in circulating VEGF concentrations.

### Summary

This Example demonstrates a potent and clinically relevant efficacy outcome of transendocardial therapy with MSCs in patients with advanced HF. Allogeneic MSCs restored flow mediated brachial artery dilatation, EPC bioactivity, and VEGF levels towards normal. As abnormalities in the vascular function of patients with CV disease is shown to be highly predictive of adverse outcomes and disease progression, targeting endothelial function is a significant therapeutic strategy. The data also reveals VEGF as a novel indicator for successful treatment of endothelial dysfunction, restoration of vascular tone, and alleviation of complications of cardiovascular disease. Subjects demonstrating improved FMD displayed reduced circulating VEGF levels, as well as higher levels of EPC-CFUs, compared to heart failure subjects demonstrating no improvement in FMD.

References:
1. Go AS, Mozaffarian D, Roger VL, et al. Heart Disease and Stroke Statistics-2014 Update: A Report From the American Heart Association. Circulation 2014;129:e28-e292.
2. Blum A. Heart failure--new insights. The Israel Medical Association journal: IMAJ 2009;11:105-11.
3. Marti CN, Gheorghiade M, Kalogeropoulos AP, Georgiopoulou VV, Quyyumi AA, Butler J. Endothelial Dysfunction, Arterial Stiffness, and Heart Failure. Journal of the American College of Cardiology 2012;60:1455-69.
4. Santiago R, Antoni B-G. Vascular dysfunction in idiopathic dilated cardiomyopathy. Nature Reviews Cardiology 2009;6:590-8.
5. Schulman IH, Zhou MS, Raij L. Interaction between nitric oxide and angiotensin II in the endothelium: role in atherosclerosis and hypertension. Journal of hypertension Supplement: official journal of the International Society of Hypertension 2006;24:S45-50.
6. Polovina MM, Potpara TS. Endothelial dysfunction in metabolic and vascular disorders. Postgraduate medicine 2014;126:38-53.
7. Zampetaki A, Kirton JP, Xu Q. Vascular repair by endothelial progenitor cells. Cardiovascular Research 2008;78:413-21.
8. Rehman J, Li J, Orschell CM, March KL. Peripheral Blood "Endothelial Progenitor Cells" Are Derived From Monocyte/Macrophages and Secrete Angiogenic Growth Factors. Circulation 2003;107:1164-9.
9. Schulman IH, Hare JM. Key developments in stem cell therapy in cardiology. Regen Med 2012;7:17-24.
10. Karantalis V, Balkan W, Schulman IH, Hatzistergos KE, Hare JM. Cell-based therapy for prevention and reversal of myocardial remodeling. Am J Physiol Heart Circ Physiol 2012;303:H256-70.
11. Williams AR, Hare JM. Mesenchymal stem cells: biology, pathophysiology, translational findings, and therapeutic implications for cardiac disease. Circulation research 2011;109:923-40.
12. Suncion VY, Schulman IH, Hare JM. Concise review: the role of clinical trials in deciphering mechanisms of action of cardiac cell-based therapy. Stem Cells Translational Medicine 2012;1:29-35.
13. Hatzistergos KE, Quevedo H, Oskouei BN, et al. Bone marrow mesenchymal stem cells stimulate cardiac stem cell proliferation and differentiation. Circulation research 2010;107:913-22.
14. Chen L, Tredget EE, Wu PY, Wu Y. Paracrine factors of mesenchymal stem cells recruit macrophages and endothelial lineage cells and enhance wound healing. PloS one 2008;3:el886.
15. Werner N, Kosiol S, Schiegl T, et al. Circulating endothelial progenitor cells and cardiovascular outcomes. N Engl J Med 2005;353:999-1007.
16. Shantsila E, Watson T, Lip GY. Endothelial progenitor cells in cardiovascular disorders. J Am Coll Cardiol 2007;49:741-52.
17. Hill JM, Zalos G, Halcox JPJ, et al. Circulating Endothelial Progenitor Cells, Vascular Function, and Cardiovascular Risk. New England Journal of Medicine 2003;348:593-600.
18. Corretti MC, Anderson TJ, Benjamin EJ, et al. Guidelines for the ultrasound assessment of endothelial-dependent flow-mediated vasodilation of the brachial artery: A report of the International Brachial Artery Reactivity Task Force. Journal of the American College of Cardiology 2002;39:257-65.
19. Werner N, Wassmann S, Ahlers P, et al. Endothelial progenitor cells correlate with endothelial function in patients with coronary artery disease. Basic research in cardiology 2007; 102:565-71.
20. Shantsila E, Watson T, Lip GYH. Endothelial Progenitor Cells in Cardiovascular Disorders. Journal of the American College of Cardiology 2007;49:741-52.
21. Schmidt-Lucke C, Rössig L, Fichtlscherer S, et al. Reduced Number of Circulating Endothelial Progenitor Cells Predicts Future Cardiovascular Events: Proof of Concept for the Clinical Importance of Endogenous Vascular Repair. Circulation 2005;111:2981-7.
22. Chin BSP, Chung NAY, Gibbs CR, Blann AD, Lip GYH. Vascular endothelial growth factor and soluble P-selectin in acute and chronic congestive heart failure. The American Journal of Cardiology 2002;90:1258-60.
23. Tsai WC, Li YH, Huang YY, Lin CC, Chao TH, Chen JH. Plasma vascular endothelial growth factor as a marker for early vascular damage in hypertension. Clinical Science 2005;109:39-43.
24. Seko Y, Fukuda S, Nagai R. Serum levels of endostatin, vascular endothelial growth factor (VEGF) and hepatocyte growth factor (HGF) in patients with acute myocardial infarction undergoing early reperfusion therapy. Clinical Science 2004;106:4.
25. Eleuteri E, Di Stefano A, Tarro Genta F, et al. Stepwise increase of angiopoietin-2 serum levels is related to haemodynamic and functional impairment in stable chronic heart failure. European Journal of Cardiovascular Prevention & Rehabilitation 2011;18:607-14.
26. Wei H, Mao Q, Liu L, et al. Changes and function of circulating endothelial progenitor cells in patients with cerebral aneurysm. Journal of neuroscience research 2011;89:1822-8.
27. Williams AR, Hare JM. Mesenchymal Stem Cells: Biology, Pathophysiology, Translational Findings, and Therapeutic Implications for Cardiac Disease. Circulation Research 2011;109:923-40.
28. Gnecchi M, Zhang Z, Ni A, Dzau VJ. Paracrine Mechanisms in Adult Stem Cell Signaling and Therapy. Circulation Research 2008;103:1204-19.
29. Caplan AI, Dennis JE. Mesenchymal stem cells as trophic mediators. Journal of Cellular Biochemistry 2006;98:1076-84.
30. Telukuntla KS, Suncion VY, Schulman IH, Hare JM. The Advancing Field of Cell-Based Therapy: Insights and Lessons From Clinical Trials. Journal of the American Heart Association 2013;2.
31. Hare JM, Traverse JH, Henry TD, et al. A randomized, double-blind, placebo-controlled, dose-escalation study of intravenous adult human mesenchymal stem cells (prochymal) after acute myocardial infarction. J Am Coll Cardiol 2009;54:2277-86.
32. Hare JM, Fishman JE, Gerstenblith G, et al. Comparison of allogeneic vs autologous bone marrow-derived mesenchymal stem cells delivered by transendocardial injection in patients with ischemic cardiomyopathy: The poseidon randomized trial. JAMA 2012;308 :2369-79.
33. Vasa M, Fichtlscherer S, Aicher A, et al. Number and Migratory Activity of Circulating Endothelial Progenitor Cells Inversely Correlate With Risk Factors for Coronary Artery Disease. Circulation Research 2001;89:e1-e7.
34. Franz Alber H, Dulak J, Frick M, et al. Atorvastatin decreases vascular endothelial growth factor in patients with coronary artery disease. Journal of the American College of Cardiology 2002;39:1951-5.
35. Yu KR, Kang KS. Aging-related genes in mesenchymal stem cells: a mini-review. Gerontology 2013;59:557-63.
36. Bustos ML, Huleihel L, Kapetanaki MG, et al. Aging Mesenchymal Stem Cells Fail to Protect Because of Impaired Migration and Antiinflammatory Response. American Journal of Respiratory and Critical Care Medicine 2014;189:787-98.
37. Efimenko A, Dzhoyashvili N, Kalinina N, et al. Adipose-Derived Mesenchymal Stromal Cells From Aged Patients With Coronary Artery Disease Keep Mesenchymal Stromal Cell Properties but Exhibit Characteristics of Aging and Have Impaired Angiogenic Potential. Stem Cells Translational Medicine 2013.
38. Asumda FZ. Age-associated changes in the ecological niche: implications for mesenchymal stem cell aging. Stem cell research & therapy 2013;4:47.
39. Chang EI, Loh SA, Ceradini DJ, et al. Age Decreases Endothelial Progenitor Cell Recruitment Through Decreases in Hypoxia-Inducible Factor 1α Stabilization During Ischemia. Circulation 2007;116:2818-29.

## Claims

1. A method of monitoring efficacy of allogeneic mesenchymal stem cell therapy for cardiomyopathy in a subject comprising
determining levels of Vascular Endothelial Growth Factor (VEGF) and endothelial progenitor cells (EPCs) in a first sample from the subject before administration of the mesenchymal stem cell therapy; and
determining levels of VEGF and EPCs in a second sample from the subject after administration of the therapy;
wherein a decreased level of VEGF in the sample and an increased level of EPCs in the second sample compared to the levels of VEGF and EPC in the first sample is indicative of effective treatment of the cardiomyopathy.

2. A method of monitoring efficacy of allogeneic mesenchymal stem cell therapy in a subject suffering from cardiomyopathy comprising determining a level of Vascular Endothelial Growth Factor (VEGF) in a sample from the subject after administration of the stem cell therapy, and comparing the level of VEGF to a predetermined criterion, wherein a decreased level of VEGF in the sample is indicative of effective treatment with allogeneic mesenchymal stem cell therapy.

3. The method according to claim 1 or claim 2, wherein the cardiomyopathy is selected from the group consisting of idiopathic dilated cardiomyopathy and ischemic cardiomyopathy.

4. The method according to claim 1 or claim 2, wherein the mesenchymal stem cell therapy includes local administration of allogeneic mesenchymal stem cells.

5. The method according to claim 4, wherein the mesenchymal stem cells are administered by transendocardial injection.

6. The method according to claim 5, wherein the mesenchymal stem cells are administered in an amount ranging from about 20 million to about 100 million cells.

7. The method according to claim 5, wherein administration of the mesenchymal stem cells results in an increase in flow-mediated vasodilation (FMD) in the subject of at least 3%.

## Patentansprüche

1. Verfahren zur Überwachung der Wirksamkeit einer allogenen mesenchymalen Stammzellentherapie für Kardiomyopathie bei einer Person, welches Folgendes umfasst:
Bestimmen der Spiegel des vaskulären endothelialen Wachstumsfaktors (VEGF - *Vascular Endothelial Growth Factor)* und der endothelialen Vorläuferzellen (EPCs - *Endothelial Progenitor Cells)* in einer ersten Probe von der Person vor einer Verabreichung der mesenchymalen Stammzellentherapie; und
Bestimmen der Spiegel des VEGF und der EPCs in einer zweiten Probe von der Person nach einer Verabreichung der Therapie;
wobei ein verminderter VEGF-Spiegel in der Probe und ein erhöhter EPC-Spiegel in der zweiten Probe im Vergleich zu den Spiegeln des VEGF und der EPCs in der ersten Probe indikativ für eine wirksame Behandlung der Kardiomyopathie sind.

2. Verfahren zur Überwachung der Wirksamkeit einer allogenen mesenchymalen Stammzellentherapie bei einer Person, die an einer Kardiomyopathie leidet, welches das Bestimmen eines Spiegels des vaskulären endothelialen Wachstumsfaktors (VEGF) in einer Probe von der Person nach einer Verabreichung der Stammzellentherapie und das Vergleichen des Spiegels des VEGF mit einem vorbestimmten Kriterium umfasst, wobei ein verminderter Spiegel des VEGF in der Probe indikativ für eine wirksame Behandlung mit einer allogenen mesenchymalen Stammzellentherapie ist.

3. Verfahren nach Anspruch 1 oder 2, wobei die Kardiomyopathie ausgewählt ist aus der Gruppe bestehend aus idiopathischer dilatativer Kardiomyopathie und ischämischer Kardiomyopathie.

4. Verfahren nach Anspruch 1 oder 2, wobei die mesenchymale Stammzellentherapie eine lokale Verabreichung von allogenen mesenchymalen Stammzellen beinhaltet.

5. Verfahren nach Anspruch 4, wobei die mesenchymalen Stammzellen durch transendokardiale Injektion verabreicht werden.

6. Verfahren nach Anspruch 5, wobei die mesenchymalen Stammzellen in einer Menge im Bereich von etwa 20 Millionen bis etwa 100 Millionen Zellen verabreicht werden.

7. Verfahren nach Anspruch 5, wobei eine Verabreichung der mesenchymalen Stammzellen in einem Anstieg in der flussvermittelten Vasodilatation (FMD - *Flow-Mediated Vasodilation)* bei der Person von mindestens 3 % resultiert.

## Revendications

1. Procédé de surveillance de l'efficacité d'une thérapie par cellules souches mésenchymateuses allogéniques pour une cardiomyopathie chez un sujet comprenant
la détermination des niveaux du facteur de croissance endothéliale vasculaire (VEGF) et des cellules progénitrices endothéliales (EPC) dans un premier échantillon du sujet avant l'administration de la thérapie par cellules souches mésenchymateuses ; et
la détermination des niveaux de VEGF et d'EPC dans un second échantillon du sujet après l'administration de la thérapie ;
dans lequel un niveau réduit de VEGF dans l'échantillon et un niveau accru d'EPC dans le second échantillon comparé aux niveaux de VEGF et d'EPC dans le premier échantillon indique un traitement efficace de la cardiomyopathie.

2. Procédé de surveillance de l'efficacité d'une thérapie par cellules souches mésenchymateuses allogéniques chez un sujet souffrant de cardiomyopathie comprenant la détermination d'un niveau de facteur de croissance endothéliale vasculaire (VEGF) dans un échantillon du sujet après l'administration de la thérapie par cellules souches, et la comparaison du niveau de VEGF à un critère prédéterminé, dans lequel un niveau réduit de VEGF dans l'échantillon indique un traitement efficace avec la thérapie par cellules souches mésenchymateuses allogéniques.

3. Procédé selon la revendication 1 ou la revendication 2, dans lequel la cardiomyopathie est sélectionnée dans le groupe constitué par une cardiomyopathie dilatée idiopathique et une cardiomyopathie ischémique.

4. Procédé selon la revendication 1 ou la revendication 2, dans lequel la thérapie par cellules souches mésenchymateuses inclut l'administration locale de cellules souches mésenchymateuses allogéniques.

5. Procédé selon la revendication 4, dans lequel les cellules souches mésenchymateuses sont administrées par injection trans-endocardique.

6. Procédé selon la revendication 5, dans lequel les cellules souches mésenchymateuses sont administrées en une quantité dans la plage d'environ 20 millions à environ 100 millions de cellules.

7. Procédé selon la revendication 5, dans lequel l'administration des cellules souches mésenchymateuses résulte en une augmentation de la vasodilatation médiée par le flux (FMD) chez le sujet d'au moins 3 %.
